Europäisches Patentamt

European Patent Office

Office européen des brevets

(11) Publication number: **0 251 494 B1**

# EUROPEAN PATENT SPECIFICATION

(45) Date of publication of patent specification: **30.09.92** (51) Int. Cl.⁵: **A61K 49/02**, A61K 43/00, A61K 47/00, A61K 47/42

(21) Application number: **87304796.3**

(22) Date of filing: **29.05.87**

(54) Therapeutic or radiodiagnostic compound.

(30) Priority: **23.06.86 US 877327**

(43) Date of publication of application:
**07.01.88 Bulletin 88/01**

(45) Publication of the grant of the patent:
**30.09.92 Bulletin 92/40**

(84) Designated Contracting States:
**AT BE CH DE ES FR GB GR IT LI LU NL SE**

(56) References cited:
**EP-A- 0 149 709**
**WO-A-87/02893**

**THE JOURNAL OF IMMUNOLOGY, vol. 125, no. 3, September 1980, The Williams & Wilkins Co. (US); G.W.PHILPOTT et al., pp. 1201-1209***

**BIOLOGICAL ABSTRACTS, vol. 83, 1987, Biological Abstracts Inc., Philadelphia, PA (US); V.S.TRUBETSKOL et al., no. 111026***

**PROCEEDINGS OF THE NATL. ACADEMY OF SCIENCE USA, vol. 80, April 1983; W.GODFREY et al., pp. 2267-2271***

(73) Proprietor: **THE BOARD OF TRUSTEES OF THE LELAND STANFORD JUNIOR UNIVERSITY**

**Stanford, California 94305(US)**

(72) Inventor: **Goodwin, David A.**
**7 Snowden Avenue**
**Atherton, CA 94025(US)**
Inventor: **Meares, Claude**
**3310 Trawler Place**
**Davis, CA 95616(US)**
Inventor: **McCall, Michael**
**513 Ridgewood Drive**
**Vacaville, CA 95688(US)**

(74) Representative: **Allard, Susan Joyce et al**
**BOULT, WADE & TENNANT, 27 Furnival Street**
**London EC4A 1PO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 93, no. 22, 01 December 1980, Columbus, OH (US); L.D.URDAL et al., no. 21047z*

THE JOURNAL OF NUCLEAR MEDICINE, no. 28, 1987; D.J.HNATOWICH et al., pp. 1294-1302*

**Description**

The present invention relates to a a therapeutic or diagnostic compound, and in particular, a radionuclide, to produce target-specific localization of the agent.

References

Chang, C.-H., et al, Biochem, Biophys Res Commun 111(3):959 (1983).
De Riemer, L.H., et al, J Med Chem 22:1019 (1979).
De Riemer, L.H., et al, J Lab Comps & Radpharm 18(10):1517 (1981).
Friguet, B., et al, J Immunol Methods 77:305 (1985).
Fujii, A.J., Antibiot 26:398 (1973).
Goodwin, D.A., et al, Nuclear Medizin 14:365 (1975).
Goodwin, D.A., et al, Seminars in Nuc Med VI:3 (1976).
Goodwin, D.A. et al, In Radiopharmaceuticals II Proceedings of the Second International Conference on Rad. N.Y., Sodd, V.J., et al, eds, pp 275-284 (1979).
Goodwin, D.A., et al, J Nuc Med 22:(9):787 (1981).
Goodwin, D.A., et al, Eur J Nuc Med 9:209 (1984).
Kohler, B., et al, Nature 256:495 (1975).
Meares, C. F., et al, Proc Natl Acad Sci (USA) 73(11):3803 (1976).
Monoclonal Antibodies, Kennett, T.J., et al, eds Plenum (1980).
Umezawa, H., Pure Appl Chem 28:665 (1970).
Wensel, T.G., et al, in Radioimaging and Radioimmunotherapy, Burchiel, S. W., et al, eds, Elsevier, p 185 (1983).

A major focus of current drug research is to improve drug targeting to internal target sites, such as to solid tumors or specific organs. The objective of drug targeting is to enhance the effectiveness of the drug by concentrating it at the target site, and minimizing its effects in non-target sites. For example, where the drug is used for therapeutic purposes, such as to treat a solid tumor, drug targeting allows more effective dosing at the target site with fewer non-tumor related side effects. Similarly, where the drug agent is a radionuclide for use in radioimaging, targeting gives enhanced contrast between the target and background areas, because of reduced background levels of the radionuclide.

Radionuclides are an important group of pharmaceutical agents for which a variety of targeting strategies have been proposed. Included in this group are radioimaging compounds, such as metal chelates of $^{111}$In, $^{67}$Ga, $^{64}$Cu, $^{99m}$Tc, $^{68}$Ga, $^{62}$Zn, $^{57}$Cu, $^{197}$Hg, $^{97}$Ru, $^{57}$Co, or $^{53}$Co, which are used to image internal sites, particularly solid tumors, by intravenous administration and systemic uptake of the chelates. Also included are radiotherapeutic agents, such as the metal chelates of $^{90}$Y, $^{197}$Hg or $^{67}$Cu, or conjugates of other radioactive elements, such as $^{131}$I which are used in treating tumors and the like, based on localized cell destruction from ionizing radiation. A related group of pharmaceutical agents are non-radioactive metal chelates such as iron, copper or ruthenium chelates, which produce cytotoxic effects through redox mechanisms, and can also potentiate the cytotoxic action of radiation on cells.

Previously, the inventors have described several novel chelate compounds which are useful for targeting radionuclides and radiosensitizing metals to internal sites, particularly solid tumors. In general, these compounds are bifunctional chelating agents which have, as one functional group, a chelating moiety capable of forming a tight complex with a metal ion, and as a second functional group, a chemically reactive moiety, such as a nitro or amine group, through which the compound can be coupled to a targeting or other molecule (Meares, 1976; Goodwin et al, 1975, 1976, 1979).

One novel class of chelate compounds which has been developed by the inventors are various ethylenediamintetraacetic acid (EDTA) chelates of bleomycin, which is an anti-tumor antibiotic which localizes within many types of tumors (Umezawa, Fujii). The bleomycin/EDTA compounds have been shown to give selective tumor localization of a variety of radionuclides, including $^{57}$Co and $^{111}$In, in solid tumors. One of the earliest of these compounds was prepared by alkylating purified bleomymin $A_2$ with a reactive bifunctional compound, such as p-bromoacetamidobenzyl-EDTA (BABE-EDTA), to link the chelate to bleomycin through a sulfonium group (DeRiemer; Goodwin, 1979, 1981; Chang).

One of the limitations which has been observed in targeting small radionuclide compounds, such as the above bleomycin/metal chelate compounds, to a target site, such as a solid tumor, is relatively low concentration of the compound at the target site. The low drug dose at the target site is is due to the rapid clearance of the compound by the kidneys, which limits the amount the compound in the bloodstream available for localization at the target site. Merely increasing the dose of the administered compound is not

3

a practical solution, since most of the radionuclides are toxic and therefore dose-limiting.

One method for increasing the concentration of a dose-limited, but rapidly cleared, target compound is to coadminister the compound in an antibody-complexed form. Because of its relatively large size, the complex is not cleared by the kidneys, but instead, is removed slowly from the bloodstream over a several day period by the reticuloendothelial system (RES). This approach has been investigated previously by the inventors, using two monoclonal antibodies (Mabs) prepared against the bifunctional indium/chelate compound L-benzyl-EDTA- $^{111}$In (LBEDTA-In). Binding studies showed that both antibodies were specific for the indium chelate, giving $K_b$ values for the indium chelate which were at least about 20 times those of the chelates of other metals. The antibodies, when coadministered with BLEDTA-$^{111}$In, increased the whole body level of BLEDTA-$^{111}$In after 24 hours between 10-30 times, presumably by retaining the BLEDTA-In compound in a tightly bound systemic form which is cleared slowly from the bloodstream.

The increased uptake of the compound in the presence of circulating anti-compound antibodies is, however, a relatively non-specific effect, since a variety of organs which were tested for $^{111}$In levels also showed significantly increased radioactivity after 24 hours. Therefore, the advantage of enhanced tumor uptake of the compound produced by coadministration of an antibody is partially offset by (1) higher background levels of radioactivity (or a therapeutic agent) in non-tumor organs, and (2), greater total patient exposure to the conjugate, e.g., greater radiation exposure in the case of a conjugate having a chelated radionuclide.

Although it may be possible to reduce these unwanted side effects by flushing the patient's bloodstream with a non-toxic or non-radioactive competing antigen, the improvement in terms of reduced exposure to the compound is not dramatic. Earlier studies conducted by the inventors showed, for example, that whole body BLEDTA-$^{111}$In levels are reduced only about 20% three hours after giving a flushing dose of Fe-EDTA. Further, the antibody-enhancement approach just described requires periods of at least several hours for significant target distribution effects; therefore, the method is not suited to radionuclides such as $^{99m}$Tc and $^{68}$Ga which have half lives of between about one to a few hours.

The system of the invention includes (a) a biotinylated compound containing the agent to be localized (b) an avidin-containing protein capable of localizing selectively at a target site when administered to a subject parenterally, and (c) a clearing agent capable of reacting with the binding protein when circulating in the bloodstream of the subject, to form a macromolecular aggregate which can be cleared by the subject's reticuloendothelial system.

The individual components of the system for use in diagnosis or therapy using the above system are included in the invention.

The present invention is further described by way of example only with reference to the accompanying drawings, in which:

Figure 1 shows the molecular structure of a biotinylated EDTA-metal chelate compound designed for use in the invention;

Figure 2 illustrates, in diagrammatic form, the steps by which a therapeutic or diagnostic compound is localized in a tissue, according to the method of the invention and in the Figure "Li" donates the liver and "K" denotes the kidneys; and

Figure 3 shows whole-body photo-emission scans of a tumor-bearing animal 3 hours (A) and 24 hours (B) after administration of a racionuclide-chelate compound.

I. Preparing the System Components

A. Biotinylated Compounds

The invention is designed for use in targeting a therapeutic or diagnostic agent at a specific internal body site, such as a solid tumor or selected organ or tissue site. The agent which is targeted is a biotinylated compound having a pharmaceutically active therapeutic or diagnostic, particularly radiomaging, moiety (the active moiety), and one or more biotin moieties which can bind specifically and with high affinity to an avidin-containing binding protein.

The active moiety of the compound is a pharmaceutically active agent, such as a drug, radionuclide, hormone, toxin, metabolite, anti-metaboilite, vitamin, enzyme-cofactor, or the like which can be derivatized to one or more biotin groups without loss of activity. Such active agents include anti-tumor agents, as exemplified by doxorubicin, cisplatin, DNA alkylating or cross-linking agents, and antimetabolites; and antimicrobial agents, such as aminoglycosides and polyene and macrolide antibiotics, such as amphotericin B. One major class of therapeutic and diagnostic agents useful in the invention are chelated metals, including chelated radionuclides useful for radioimaging, such as $^{111}$In, $^{67}$Ga, $^{64}$Cu, $^{99m}$Tc, $^{68}$Ga, $^{62}$Zn,

EP 0 251 494 B1

$^{67}$Cu, $^{197}$Hg, $^{97}$Ru, $^{57}$Co, or $^{53}$Co; chelated radionuclides useful for tumor therapy, such as $^{90}$Y, $^{197}$Hg or $^{67}$Cu; and a radio-sensitizing chelated metals, such as chelated iron, copper or ruthenium.

The method of the invention requires that the biotinylated compound be sufficiently small and soluble when administered parenterally that it can be cleared rapidly from the kidneys. Typically molecules with molecular weights less than 50,000 daltons, and preferably less than 10,000 daltons, and which exist predominantly in monomolecular form in serum generally satisfy these requirements.

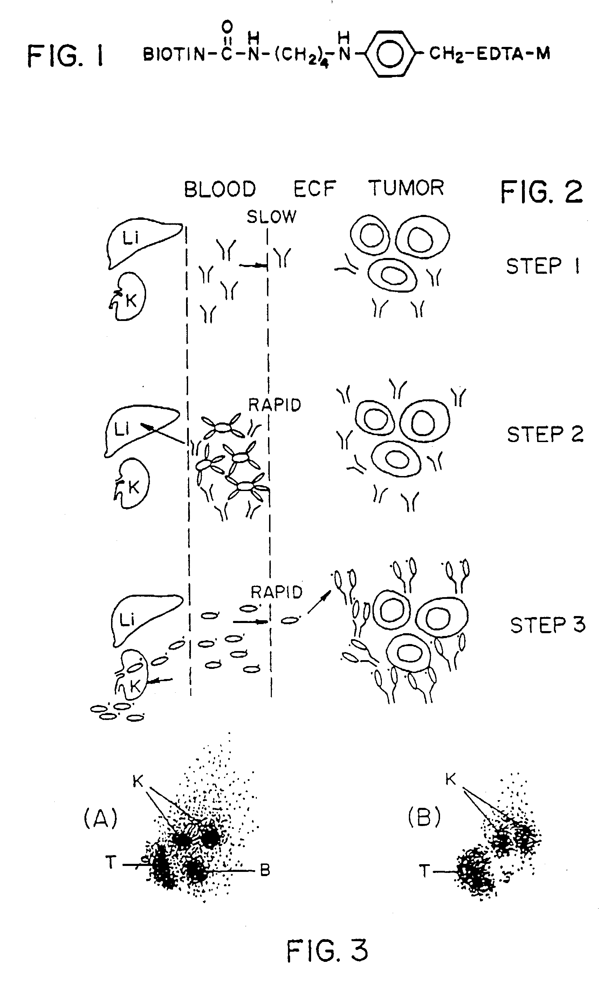

Methods for derivatizing small target molecules with one or more biotin groups follow well-known coupling procedures for biotinylating compounds at reactive chemical sites, such as carboxyl, amino, nitro, hydroxyl, aldehyde, and sulhydryl sites. In one preferred method, the N-hydroxysuccinimide ester of biotin (biotin-NHS), a commercially available compound, is reacted directly with an amine group in the targeting compound, for coupling through an amide linkage. The amine group may itself may be added to the target compound as a spacer arm, which is also joined to the target compound by standard coupling techniques. By way of illustration, the biotinylated EDTA compound illustrated in Figure 1 was formed by reacting a putrescein (1,4-butanediamine) with p-isothiocyanato benzyl EDTA, to couple the putrescein spacer arm to the bifunctional EDTA compound. Derivatization with biotin-NHS then yielded the desired biotinylated compound. Other coupling methods suitable for producing biotinylated target molecules are available and known to those in the art.

Biotinylated compounds containing two or more biotin groups, to give enhanced binding to avidin or streptavidin, may be prepared by the same general methods discussed above. In derivatizing an active agent with multiple biotin groups, the individual groups can be attached to different sites on an active agent, or may be attached at different positions on a single spacer arm attached to the active agent.


B. Avidin-Containing Binding Proteins


The avidin-containing binding protein in the system of the invention serves both as a targeting agent capable of localizing specifically at an internal target site, and as a binding agent, for binding the biotinylated compound to the target site.

The inventors have earlier described a targeting system in which the binding protein is an antibody capable of binding specifically to a hapten-derivatized compound. Whereas antibodies offer the advantage that they can be prepared against a wide variety of epitopes, avidin has the advantage of a very high binding affinity (about $10^{15}$ M$^{-1}$) for the epitope biotin, as compared to binding affinities of between $10^9$ to $10^{12}$ for hapten/antibody pairs. The advantage of the high binding constant in the present invention is that the invention can be practiced using much smaller quantities of injected binding protein. Specifically, the amount of avidin which need be injected can be several orders of magnitude smaller than the amount of antibody needed to produce equivalent levels of bound target compound at the target site. Accordingly, immune reaction problems which may arise by parenteral administration of relatively large doses of foreign protein are greatly minimized.

Avidin, an egg protein, and streptavidin are commercially available in purified form suitable for use in human therapeutics. These proteins can be derivatized to antibody or antibody fragment molecules by known coupling methods, which include condensation and cross-linking reactions. The monoclonal antibody/streptavidin conjugate described in Example 2 was formed using the cross-linking reagents 2-imminothiolane and succinimidyl 4-(N-maleimidomethyl)cyclohexane-1-carboxylate, according to known methods. Other coupling agents, such as soluble carbodiimides, and di-N-hydroxysuccinimide compounds may also be used.

Considering the targeting function of the binding protein, the invention relies on the ability of the protein, when administered parenterally, to localize selectively from the bloodstream to the target site. For efficient localization, this requires that the protein (a) have a relatively long circulating half life in the bloodstream, and (b) be capable of accumulating in the target organ. In terms of size requirements, avidin, streptavidin, and antibodies or antibody fragments derivatized with either is large enough to prevent rapid renal clearance, but not so large as to promote rapid clearance by the RES. Binding proteins, including various types of hybrid proteins discussed below, in the size range between 50-500,000 daltons are most suitable.

The targeting capability of the binding protein may be based on the ability of the protein to bind specifically to target site antigens, or on protein size or membrane permeability characteristics, or to a combination of these factors. Most target sites, including tumor target sites contain tissue-specific surface antigens against which the binding protein can be directed, and antibodies specific against a variety of normal and malignant tissues, such as used in Example 2, have been reported. The antibody may be an intact serum, or preferably monoclonal, antibody specific against target tissue antigens, or an antibody fragment, such as the F(ab')$_2$ or Fab fragment produced by enzymatic cleavage of intact antibody. In the

5

antibody-avidin conjugate, the antibodies or antibody fragments in the hybrid protein would function is tissue targeting, while the avidin moiety would provide high affinity binding for biotin-labelled compounds which are targeted to the site.

In another embodiment which is suitable for tumor targeting, selective protein localization in the tumor is based on the ability of the protein to penetrate selectively the more permeable, i.e., leaky, capillaries which normally are associated with solid tumors. The advantage of this approach is greater simplicity in preparing the binding protein.

Examples 1 and 2 below illustrate tumor targeting with streptavidin alone (Example 1), and streptavidin conjugated with an anti-tumor monoclonal antibody.

## C. Clearing Agent

The clearing agent in the system of the invention functions to bind to and cross-link binding proteins which are circulating in the bloodstream of the treated individual, to promote rapid clearance of the binding protein aggregates by the RES. The agent is preferably large enough to avoid rapid renal clearance, and contains sufficient multivalency to cross-link and aggregate circulating binding proteins. Therefore, the protein preferably has a molecular weight of 50,000 daltons or more, and is derivatized with biotin at a mole ratio of about 1:4 protein:biotin or higher. Here it is noted that protein aggregation by the clearing agent also requires at least two avidin molecules on each binding protein.

One preferred clearing agent, for use in humans, is a human protein derivatized with multiple biotin groups. For example, human transferrin can be biotinylated by reaction with biotin in the presence of a suitable condensing agent, such as a soluble carbodiimide, or by reaction with the above biotin-NHS reagent.

## II. Therapeutic and Radiodiagnostic Methods

### A. Localizing the Binding Protein

The method of the invention is designed for targeting a therapeutic or radiodiagnostic agent to a selected internal body site, such as a tumor region, an internal organ, or some other specific tissue region.

As a first step in the method the binding protein is administered parenterally, i.e., into the bloodstream, preferably by intravenous (IV) administration. From here, the protein slowly passes from the blood, through the extracellular fluid (ECF), and into the body tissues, including the target site, accumulating preferentially in the tumor site because of the targeting feature of the protein. This initial step is illustrated at the top in Figure 2, which shows slow passage of an avidin-labeled antibody binding protein from the bloodstream (dotted-line column) through the ECF into a tumor site.

According to an important advantage of the method, the binding protein is delivered in non-complexed form, i.e., without bound biotinylated compound so that the treated individual is not exposed to the compound during the extended period of protein localization at the tumor site. The amount of binding protein which is injected is calculated to yield a selected concentration of the biotinylated compound at the target site, in the final targeting step. This amount will depend on several factors which include: (a) the relative degree of localization of the binding protein in target and non-target regions of the body, (b) the persistence of the binding protein in the blood and, most importantly, (c) the binding constant of the binding protein, when localized at the target site, for the epitopic compound.

The optimal amount of binding protein which is administered can be approximately determined by combining known binding constant data with empirical studies on animal model systems. Studies conducted in support of the present invention indicate that effective targeting of a biotinylated EDTA compound required an initial avidin or streptavidin doses which are substantially less than the 10-100 mg dose required for achieving the same level of localized chelate using an anti-chelate antibody as the prelocalizing agent.

The administered binding protein, which has a circulating half life in the blood of one to several days, is allowed to localize in the target organ over period of typically about 1-4 days. During this period the binding protein is slowly taken up from the blood by the target tissue, as well as other tissues. In the case of tumors, binding protein accumulation at the target site is enhanced because of the relatively greater leakiness of the capillaries which supply the tumor. As mentioned earlier, tumor localization can be based solely on preferential protein (e.g., avidin or streptavidin) leakage into the tumor. This mechanism is illustrated in the targeting methods illustrated in Examples 1 and 2, which both show accumulation of an avidin binding protein in tumor regions 24 hours after intravenous administration.

B. Clearing the Binding Agent

In the second step of the method, the circulating binding protein is cleared rapidly from the bloodstream, to reduce total blood levels of the protein severalfold, without appreciably effecting the levels of binding protein which have accumulated in the target. This step, which is illustrated in the center frame of Figure 2, is based on the formation in the bloodstream of aggregates of binding proteins and clearing agent, and rapid clearance of these aggregates by the RES. In the figure, the aggregates formed in the bloodstream are shown being removed by the liver, the principle site of uptake by the RES. Because of its rapid removal, the clearing agent does not accumulate appreciably outside the bloodstream, and therefore has little effect on the disposition of binding protein already localized outside the bloodstream.

The amount of clearing agent which is administered is preferably in a molar ratio of between about 1:5-5:1 with respect to the quantity of binding protein calculated to be the bloodstream of the treated individual at the time of the clearing step. Experiments conducted in support of the application indicate that the amount of binding protein present in the bloodstream 24 hours after iv administration is typically between 15-25% of the total amount administered.

The total time allowed for clearance of the binding protein, i.e., before the epitopic compound is administered, may be as short as 15 minutes, but typically ranges from 1-4 hours. In the study reported in Example 2, blood levels were reduced about over 40 fold with the chase, as measured by the amount of radiolabelled biotinylated chelate taken up by the blood before and after the clearing step.

C. Uptake of the Biotinylated Compound

In the final targeting step, the biotinylated compound is administered parenterally, and preferably intravenously, for selective uptake of the localized binding protein. This step, which is illustrated at the bottom in Figure 2, involves rapid uptake of the compound by the localized binding protein, in competition with rapid clearance from the bloodstream by the kidneys.

The amount of biotinylated compound which is administered is calculated to produce a desired concentration of compound at the target site shortly after compound injection, usually within 1-4 hours after injection. As with the binding protein, the optimal dosage can be calculated approximately from studies on a model animal system combined with the known binding affinity of the binding protein for the compound.

A number of studies on the tumor localization of biotinylated chelate-radionuclide compounds have been carried out in support of the invention, and some of these studies are detailed in Examples 1 and 2. With both streptavidin and antibody-streptavidin conjugate binding proteins, tumor/blood ratios increased severalfold with administration of the chase.

In a related study, detailed in Example 3, a monoclonal antibody (not conjugated to avidin) specific against an non-biotinylated EDTA chelate was administered, followed by a chase after 20 hours with transferrin-labelled chelate. One hour later, chelate-$^{57}$Co was given, and allowed to localize for three hours. Whole-body radioimmaging of the treated animal gave the results seen in Figure 3A, which shows localization of the label in the kidneys (K), bladder (B) and flank tumor (T). The distribution of the label in the tumor, blood and other tissues three hours after label injection is shown in Table 1 in Example 3. The data show high tumor-to-organ ratios for blood, and other internal organs. The higher levels of radiolabel in the kidneys seen in the present study is due presumably to slower clearance of the radiolabeled compound from the kidneys.

Figure 3B shows the same animal imaged in Figure 3A, but imaged 24 hours after compound administration. The major difference in the longer-term imaging is an absence of label in the bladder. Good tumor imaging with low background is still achieved. The example 3 study indicates the enhancement in tumor imaging that can be achieved by the method of the invention, but differs from the invention in that the antibody (binding protein) levels which must be administered are substantially higher than those needed in the present invention to achieve sufficient concentration of the targeting compound in the target site.

From the foregoing, it can be appreciated how various objects and features of the invention are met. The method provides selective localization of a therapeutic or radioimaging compound, based on accumulation of a target-specific binding protein at the selected target site. This feature allows for improved therapeutic effect with reduced side effects for therapeutic agents, and improved imaging with reduced background levels, particularly background levels due to circulating radionuclide.

Because the localization of the compound occurs shortly after compound injection, e.g., 1-4 hours, radionuclides with half lives of about 1-6 hours can be used for radioimaging. In particular, the method allows both $^{99m}$Tc, whose half life is about 6 hours, and $^{68}$Ga, whose half life is 68 minutes, to be used at relatively low radioactivity levels for radioimaging of tumors or other internal target sites. The particular

advantage of $^{68}$Ga as a radioimaging agent is its use in photon emission tomography, an imaging technique that allows quantitation of localized radiolabel and resolution down to 5 mm. Heretofore, the large amount of radionuclide needed for imaging required on on-site cyclotron. With the present invention, the ability to localize large quantities of the radionuclide in an hour or less is compatible with radionuclide amounts which can be produced with a much less expensive $^{68}$Ga generator.

The rapid clearance of non-localized compound by the kidneys means that the toxicity associated with the compound in systemic form can be reduced substantially. In the case of $^{111}$In radioimaging, for example, it has been necessary heretofore to attach the metal to a circulating protein, to achieve high levels of the radionuclide at a localized site. This approach is limited by the amount of protein-conjugated metal which can be safely administered due to high levels of the radionuclide in the bloodstream over a several day period. In the present invention, most of the non-localized radionuclide is cleared in a few hours. This advantage also applies to therapeutic drugs, such as anti-tumor drugs, which must be given in large doses to achieve an effective drug level at the target site. Drug treatment has been limited heretofore, for many anti-tumor drugs, by serious side effects associated with large dose levels. In the present invention, the specific binding of the drug at the target sites, due to the presence there of concentrated binding protein, allows therapeutically effective drug concentrations to be achieved at the tumor site with smaller doses of injected drug.

According to another important feature of the invention, the avidin-containing binding protein can be administered in relatively low doses due to its high binding affinity for the biotinylated target compound. Therefore the risk of immune reaction to the administered binding protein is substantially less than where the binding protein is an unconjugated antibody, which must be administered in substantially greater dosage. Further, the cost of treatment materials is reduced.

The following examples illustrate the preparation and use of specific embodiments of the invention, but are in no way intended to limit the scope of the invention.

## Example 1

### Biodistribution of In-11 Biotin Compound

Biotin was coupled to p-isothicyanato EDTA through a putrescein spacer, as described above, and the biotinylated chelate compound was labeled with In-111, according to standard methods. The animals retained about 6% of the total radioactivity 6 hours after intravenous injection, and about 2%, 24 hours after injection. When the In-111 compound (0.03 nmoles) was complexed with avidin (0.1 nmole), and administered intravenously, 70% was retained after 24 hours, but was localized mostly in the liver (42%/g liver; 0.03%/g blood%). When streptavidin was substituted, 75% was retained after 24 hours, but stayed in circulation to a much greater extent (10%/g liver; 6%/g blood).

BALB/c mice with KHJJ tumors were pretargeted with 0.25 nmole steptavidin by intravenous injection. At 20 hours, an iv chase of biotinylated human transferrin was given, followed at 21 hours with 0.016 nmole of the above biotinylated EDTA-In-111 compound. The biodistribution of the In-111 compound, as measured 3 hours after compound administration, showed a tumor uptake level of 0.45% dose/g, and a tumor/blood ratio of 4.74. In the absence of chase (the biotinylated human transferrin given at 20 hours), the tumor/blood ratio was 1.52.

The data above indicate that (a) good tumor targeting can be achieved by chelate binding to prelocalized streptavidin, and (b) the removal of blood streptavidin by a chase significantly reduces In-111 background due to blood levels of the bound chelate complex.

## Example 2

### Targeting with Chimeric Antibodies

Mouse monoclonal antibody SIC5, an IgG2a antibody, which is anti-idiotypic to surface IgM on mouse B cell lymphoma 38C-13 was covalently linked to streptavidin using the bifunctional crosslinking reagents 2-imminothiolane and and succinimidyl 4-(N-maleimidomethyl)cyclohexane-1-carboxylate, both obtained from commercial sources. Biotinylated EDTA complexed with In-111 was prepared as above.

The bifunctional chimeric antibody was injected intravenously in C3H mice with 38C-13 flank tumors, and allowed to localize for 20 hours, at which time biotinylated human transferrin was given as a chase. One hour later, when the chase had largely cleared the blood of the antibody, the animals were each injected IV with the biotinylated-EDTA-In-111 compound, and organ distribution of radioactivity was measured within 1-3 hours, when renal clearance had lowered the background levels of In-111 sufficiently. Control animals were similarly treated but without receiving the chase and/or antibody, and with covalently labeled In-111 chelate conjugates of the SIC5 antibody.

With covalent In-111 antibody, background was high in all organs, especially liver, spleen, lymph nodes and blood. In pretargeted animals (inital antibody administration), background levels were also high, especially in blood. The chase removed most of the blood background with improved tumor/blood ratios (greater than 6 fold increase).

Example 3

Tumor Imaging

Three A Balb/c mice prepared with a KHJJ adenocarcinoma tumor implants in the flank, were administered 100 mg of monoclonal antibody specific against an EDTA conjugate. Twenty hours after antibody administration, the animals were given a chase of the transferrin/EDTA-conjugate clearing agent, and one hour later (21 hours after antibody administration), the animal received an injection of the EDTA conjugate complexed with In-111. Three hours later, a whole-body scan, using computer digitation of the whole-body image, was performed on the animals. A representative image obtained for one of the animals in shown in Figure 3A. The image shows a concentration of radiolabel in the kidneys (K), bladder (B), and the tumor region (T). 16% of the $^{111}$In administered remained after 3 hours. Two of the animals were sacrificed at this time and examined for $^{111}$In levels in the blood, tumor, and other tissues listed in Table 1 below. As seen from the table, the regimen gave good tumor uptake, and high tumor to blood and low tumor to kidney ratios, consistent with (a) localization of radionuclide in the tumor, and (b) rapid clearance of the radionuclide from the blood by the kidneys.

## TABLE 1

| Organ | % Dose Per gm Organ | Tumor/ Organ |
|---|---|---|
| Blood | 0.71 | 5.55 |
| Heart | 0.40 | 9.85 |
| Lungs | 1.01 | 3.91 |
| Liver | 1.24 | 3.20 |
| Spleen | 0.39 | 10.11 |
| Kidneys | 37.78 | 0.11 |
| Tumor | 3.95 | 1.00 |
| Muscle | 0.21 | 18.52 |
| Bone | 1.06 | 3.73 |
| Skin | 0.05 | 4.64 |
| Gut | 1.17 | 3.39 |

A similar whole-body scan was made 24 hours later (48 hours after the antibody administration). The image obtained, shown in Figure 3B, is similar to the earlier scan, but shows relative absence of label in the bladder. 14% of the original $^{111}$material injected was present.

**Claims**
**Claims for the following Contracting States : BE, CH, DE, FR, GB, IT, LI, LU, NL, SE**

1. A pharmaceutical composition having components which localise a diagnostic or therapeutic agent at an internal target site in a subject when the components are sequentially administered parenterally to the subject, said components comprising

an avidin-containing binding protein capable of localising selectively at the target site when administered parenterally to the subject;

a clearing agent comprising a biotinylated protein, said protein having a molecular weight of at least 50,000 daltons and having a protein:biotin ratio of at least 1:4 capable of reacting with said binding protein when circulating in the bloodstream of the subject and forming a macromolecular aggregate which is cleared by the subject's particular reticuloendothelial system; and

a biotinylated compound containing the agent derivatized with biotin.

2. A pharmaceutical composition as claimed in claim 1, wherein the binding protein is avidin or streptavidin.

3. A pharmaceutical composition as claimed in claim 1, wherein the binding protein is an antibody conjugated to avidin or streptavidin.

4. A pharmaceutical composition as claimed in any one of claims 1 to 3, for use in delivering a radionuclide to the target site, wherein said biotinylated compound is a biotinylated chelate compound complexed with a metal radionuclide ion to form a stable metal chelate complex.

5. A pharmaceutical composition as claimed in claim 4, wherein the chelate compound is a metal chelate of 1-phenyl or 1-benzyl EDTA.

6. A pharmaceutical composition as claimed in claim 5, wherein the chelate compound is a metal chelate having a thiobutane spacer arm linked to the benzyl moiety.

7. A pharmaceutical composition as claimed in any one of claims 4 to 6, for use in treating a solid tumor and wherein the metal chelate is a chelate of $^{90}$Y, $^{197}$Hg or $^{67}$Cu, or for use in radiosensitizing a body tumor and wherein the metal chelate is a chelate of iron, copper or ruthenium.

8. A pharmaceutical composition as claimed in any of claims 4 to 6, which is for use in radioimaging a body tumor and wherein the metal chelate is a chelate of $^{111}$In, $^{67}$Ga, $^{64}$Cu, $^{99m}$Tc, $^{68}$Ga, $^{62}$Zn, $^{67}$Cu, $^{197}$Hg, $^{97}$Ru, $^{57}$Co, of $^{53}$Co.

9. A pharmaceutical composition as claimed in any one of the preceding claims, wherein the binding protein is selectively permeable across the walls of capillaries which supply blood to a tumor.

**Claims for the following Contracting States : AT, ES, GR**

1. A method of producing a pharmaceutical composition having components which localise a diagnostic or therapeutic agent at an internal target site in a subject when the components are sequentially administered parenterally to the subject which comprises the following steps:

providing an avidin-containing binding protein capable of localising selectively at the target site when administered parenterally to the subject;

providing a clearing agent comprising a biotinylated protein, said protein having a molecular weight of at least 50,000 daltons and having a protein:biotin ratio of at least 1:4 capable of reacting with said binding protein when circulating in the bloodstream of the subject and forming a macromolecular aggregate which is cleared by the subject's particular reticuloendothelial system; and

providing a biotinylated compound containing the agent derivatized with biotin, such that said pharmaceutical composition is formed after said sequential administration of the components.

2. A method as claimed in claim 1, wherein the binding protein is avidin or streptavidin.

3. A method as claimed in claim 1, wherein the binding protein is an antibody conjugated to avidin or streptavidin.

4. A method as claimed in any one of claims 1 to 3, for use in delivering a radionuclide to the target site, wherein said biotinylated compound is a biotinylated chelate compound complexed with a metal radionuclide ion to form a stable metal chelate complex.

5. A method as claimed in claim 4, wherein the chelate compound is a metal chelate of 1-phenyl or 1-benzyl EDTA.

6. A method as claimed in claim 5, wherein the chelate compound is a metal chelate having a thiobutane spacer arm linked to the benzyl moiety.

7. A method as claimed in any one of claims 4 to 6, for use in treating a solid tumor and wherein the metal chelate is a chelate of $^{90}Y$, $^{197}Hg$ or $^{67}Cu$, or for use in radiosensitizing a body tumor and wherein the metal chelate is a chelate of iron, copper or ruthenium.

8. A method as claimed in any of claims 4 to 6, which is for use in radioimaging a body tumor and wherein the metal chelate is a chelate of $^{111}In$, $^{67}Ga$, $^{64}Cu$, $^{99m}Tc$, $^{68}Ga$, $^{62}Zn$, $^{67}Cu$, $^{197}Hg$, $^{97}Ru$, $^{57}Co$, of $^{53}Co$.

9. A method as claimed in any one of the preceding claims, wherein the binding protein is selectively permeable across the walls of capillaries which supply blood to a tumor.

**Patentansprüche**
**Patentansprüche für folgende Vertragsstaaten : BE, CH, DE, FR, GB, IT, LI, LU, NL, SE**

1. Pharmazeutische Zusammensetzung mit Komponenten, die ein diagnostisches oder therapeutisches Mittel an einem Zielort des Körpers in einem Patienten lokalisieren, wenn die Komponenten dem Patienten aufeinanderfolgend parenteral verabreicht werden, wobei die Komponenten umfassen:
ein Avidin-haltiges Bindeprotein, das zur selektiven Lokalisierung am Zielort in der Lage ist, wenn es dem Patienten parenteral verabreicht wird;
ein Reinigungsmittel, das ein biotinyliertes Protein enthält, wobei das Protein ein Molekulargewicht von mindestens 50.000 Da und ein Protein:Biotin-Verhältnis von mindestens 1:4 aufweist, das bei Zirkulation im Blutstrom des Patienten mit dem Bindeprotein reagieren und ein makromolekulares Aggregat bilden kann, das durch das jeweilige retikuloendotheliale System des Patienten ausgeschieden wird; und
eine biotinylierte Verbindung, die das mit Biotin derivatisierte Mittel enthält.

2. Pharmazeutische Zusammensetzung nach Anspruch 1, worin das Bindeprotein Avidin oder Streptavidin ist.

3. Pharmazeutische Zusammensetzung nach Anspruch 1, worin das Bindeprotein ein Antikörper ist, der mit Avidin oder Streptavidin konjugiert ist.

4. Pharmazeutische Zusammensetzung nach einem der Ansprüche 1 bis 3 zur Verwendung beim Transport eines Radionuclids zum Zielort, worin die biotinylierte Verbindung eine biotinylierte Chelatverbindung ist, die mit einem Radionuclidmetallion komplexiert ist, um einen stabilen Metall-Chelat-Komplex zu bilden.

5. Pharmazeutische Zusammensetzung nach Anspruch 4, worin die Chelatverbindung ein Metallchelat von 1-Phenyl- oder 1-Benzyl-EDTA ist.

6. Pharmazeutische Zusammensetzung nach Anspruch 5, worin die Chelatverbindung ein Metallchelat mit einem Thiobutanspacerarm, verknüpft mit der Benzylgruppierung, ist.

7. Pharmazeutische Zusammensetzung nach einem der Ansprüche 4 bis 6 zur Verwendung bei der Behandlung eines festen Tumors und worin das Metallchelat ein Chelat von $^{90}Y$, $^{197}Hg$ oder $^{67}Cu$ ist, oder zur Verwendung bei der Radiosensibilisierung eines Körpertumors und worin das Metallchelat ein Chelat von Eisen, Kupfer oder Ruthenium ist.

8. Pharmazeutische Zusammensetzung nach einem der Ansprüche 4 bis 6, die zur Verwendung bei der Radioabbildung eines Körpertumors ist, und worin das Metallchelat ein Chelat von $^{111}In$, $^{67}Ga$, $^{64}Cu$,

$^{99m}$Tc, $^{68}$Ga, $^{62}$Zn, $^{67}$Cu, $^{197}$Hg, $^{97}$Ru, $^{57}$Co oder $^{53}$Co ist.

9. Pharmazeutische Zusammensetzung nach einem der vorhergehenden Ansprüche, worin das Bindeprotein die Wände von Kapillaren, die Blut zu einem Tumor liefern, selektiv durchdringen kann.

**Patentansprüche für folgende Vertragsstaaten : AT, ES, GR**

1. Verfahren zur Herstellung einer pharmazeutischen Zusammensetzung mit Komponenten, die ein diagnostisches oder therapeutisches Mittel an einem Zielort des Körpers in einem Patienten lokalisieren, wenn die Komponenten dem Patienten aufeinanderfolgend parenteral verabreicht werden, umfassend die folgenden Schritte:
Bereitstellen eines Avidin-haltigen Bindeproteins, das selektiv am Zielort lokalisiert werden kann, wenn es dem Patienten parenteral verabreicht wird;
Bereitstellen eines Reinigungsmittels, das ein biotinyliertes Protein enthält, wobei das Protein ein Molekulargewicht von mindestens 50.000 Da und ein Protein:Biotin-Verhältnis von mindestens 1:4 aufweist, das bei Zirkulation im Blutstrom des Patienten mit dem Bindeprotein reagieren und ein makromolekulares Aggregat bilden kann, das durch das jeweilige retikuloendotheliale System des Patienten ausgeschieden wird; und
Bereitstellen einer biotinylierten Verbindung, die das mit Biotin derivatisierte Mittel enthält, so daß die pharmazeutische Zusammensetzung nach aufeinanderfolgender Verabreichung der Komponenten gebildet wird.

2. Verfahren nach Anspruch 1, worin das Bindeprotein Avidin oder Streptavidin ist.

3. Verfahren nach Anspruch 1, worin das Bindeprotein ein Antikörper ist, der mit Avidin oder Streptavidin konjugiert ist.

4. Verfahren nach einem der Ansprüche 1 bis 3 zur Verwendung beim Transport eines Radionuclids zum Zielort, worin die biotinylierte Verbindung eine biotinylierte Chelatverbindung ist, die mit einem Radionuclidmetallion komplexiert ist, um einen stabilen Metall-Chelat-Komplex zu bilden.

5. Verfahren nach Anspruch 4, worin die Chelatverbindung ein Metallchelat von 1-Phenyl- oder 1-Benzyl-EDTA ist.

6. Verfahren nach Anspruch 5, worin die Chelatverbindung ein Metallchelat mit einem Thiobutanspacerarm, verknüpft mit der Benzylgruppierung, ist.

7. Verfahren nach einem der Ansprüche 4 bis 6 zur Verwendung bei der Behandlung eines festen Tumors und worin das Metallchelat ein Chelat von $^{90}$Y, $^{197}$Hg oder $^{67}$Cu ist, oder zur Verwendung bei der Radiosensibilisierung eines Körpertumors und worin das Metallchelat ein Chelat von Eisen, Kupfer oder Ruthenium ist.

8. Verfahren nach einem der Ansprüche 4 bis 6, die zur Verwendung bei der Radioabbildung eines Körpertumors ist, und worin das Metallchelat ein Chelat von $^{111}$In, $^{67}$Ga, $^{64}$Cu, $^{99m}$Tc, $^{68}$Ga, $^{62}$Zn, $^{67}$Cu, $^{197}$Hg, $^{97}$Ru, $^{57}$Co oder $^{53}$Co ist.

9. Verfahren nach einem der vorhergehenden Ansprüche, worin das Bindeprotein die Wände von Kapillaren, die Blut zu einem Tumor liefern, selektiv durchdringen kann.

**Revendications**
**Revendications pour les Etats contractants suivants : BE, CH, DE, FR, GB, IT, LI, LU, NL, SE**

1. Composition pharmaceutique comprenant des constituants qui localisent un agent de diagnostic ou un agent thérapeutique au niveau d'un site interne choisi comme cible chez un sujet lorsque les constituants sont administrés successivement par voie parentérale au sujet, lesdits constituants comprenant
une protéine de liaison contenant de l'avidine, capable de se localiser sélectivement au niveau du site servant de cible lors de son administration parentérale au sujet ;

12

EP 0 251 494 B1

un agent de dissipation comprenant une protéine biotinylée, ladite protéine ayant un poids moléculaire d'au moins 50 000 daltons et ayant un rapport protéine:biotine d'au moins 1:4, capable de réagir avec ladite protéine de liaison lors de la circulation dans le courant sanguin du sujet et formant un agrégat macromoléculaire qui est dissipé par le système réticulo-entothélial particulier du sujet ; et

un composé biotinylé contenant l'agent transformé en dérivé avec la biotine.

2. Composition pharmaceutique suivant la revendication 1, dans laquelle la protéine de liaison est l'avidine ou la streptavidine.

3. Composition pharmaceutique suivant la revendication 1, dans laquelle la protéine de liaison est un anticorps conjugué à l'avidine ou à la streptavidine.

4. Composition pharmaceutique suivant l'une quelconque des revendications 1 à 3, destinée à être utilisée pour délivrer un radionucléide au site choisi comme cible, dans laquelle le composé biotinylé est un chélate biotinylé complexé avec un radionucléide consistant en un ion métallique pour former un complexe de chélate métallique stable.

5. Composition pharmaceutique suivant la revendication 4, dans laquelle le chélate est un chélate métallique de 1-phényl- ou 1-benzyl-EDTA.

6. Composition pharmaceutique suivant la revendication 5, dans laquelle le chélate est un chélate métallique comprenant un bras intercalaire thiobutane lié au groupement benzyle.

7. Composition pharmaceutique suivant l'une quelconque des revendications 4 à 6, destinée à être utilisée dans le traitement d'une tumeur solide et dans laquelle le chélate métallique est un chélate de $^{90}Y$, $^{197}Hg$ ou $^{67}Cu$, ou bien destinée à être utilisée dans la radiosensibilisation d'une tumeur somatique et dans laquelle le chélate métallique est un chélate de fer, de cuivre ou de ruthénium.

8. Composition pharmaceutique suivant l'une quelconque des revendications 4 à 6, qui est destinée à être utilisée en radio-imagerie d'une tumeur somatique et dans laquelle le chélate métallique est un chélate de $^{111}In$, $^{67}Ga$, $^{64}Cu$, $^{99m}Tc$, $^{68}Ga$, $^{62}Zn$, $^{67}Cu$, $^{197}Hg$, $^{97}Ru$, $^{57}Co$ ou $^{53}Co$.

9. Composition pharmaceutique suivant l'une quelconque des revendications précédentes, dans laquelle la protéine de liaison peut passer sélectivement à travers les parois des capillaires qui amènent le sang à une tumeur.

**Revendications pour les Etats contractants suivants : AT, ES, GR**

1. Procédé de production d'une composition pharmaceutique comprenant des constituants qui localisent un agent de diagnostic ou un agent thérapeutique au niveau d'un site interne choisi comme cible chez un sujet lorsque les constituants les constituants sont administrés successivement par voie parentérale au sujet, qui comprend les étapes suivantes :

à utiliser une protéine de liaison contenant de l'avidine, capable de se localiser sélectivement au niveau du site servant de cible lors de son administration parentérale au sujet ;

à utiliser un agent de dissipation comprenant une protéine biotinylée, ladite protéine ayant un poids moléculaire d'au moins 50 000 daltons et ayant un rapport protéine:biotine d'au moins 1:4, capable de réagir avec ladite protéine de liaison lors de la circulation dans le courant sanguin du sujet et formant un agrégat macromoléculaire qui est dissipé par le système réticulo-entothélial particulier du sujet ; et

à utiliser un composé biotinylé contenant l'agent transformé en dérivé avec la biotine, de telle sorte que ladite composition pharmaceutique soit formée après administration successive des constituants.

2. Procédé suivant la revendication 1, dans lequel la protéine de liaison est l'avidine ou la streptavidine.

3. Procédé suivant la revendication 1, dans lequel la protéine de liaison est un anticorps conjugué à l'avidine ou à la streptavidine.

4. Procédé suivant l'une quelconque des revendications 1 à 3, destiné à être utilisé pour délivrer un radionucléide au site choisi comme cible, dans lequel le composé biotinylé est un chélate biotinylé

13

complexé avec un radionucléide consistant en un ion métallique pour former un complexe de chélate métallique stable.

5. Procédé suivant la revendication 4, dans lequel le chélate est un chélate métallique de 1-phényl- ou 1-benzyl-EDTA.

6. Procédé suivant la revendication 5, dans lequel le chélate est un chélate métallique comprenant un bras intercalaire thiobutane lié au groupement benzyle.

7. Procédé suivant l'une quelconque des revendications 4 à 6, destiné à être utilisé dans le traitement d'une tumeur solide et dans lequel le chélate métallique est un chélate de $^{90}$Y, $^{197}$Hg ou $^{67}$Cu, ou bien destiné à être utilisé dans la radiosensibilisation d'une tumeur somatique et dans lequel le chélate métallique est un chélate de fer, de cuivre ou de ruthénium.

8. Procédé suivant l'une quelconque des revendications 4 à 6, qui est destiné à être utilisé en radioimagerie d'une tumeur somatique et dans lequel le chélate métallique est un chélate de $^{111}$In, $^{67}$Ga, $^{64}$Cu, $^{99m}$Tc, $^{68}$Ga, $^{62}$Zn, $^{67}$Cu, $^{197}$Hg, $^{97}$Ru, $^{57}$Co ou $^{53}$Co.

9. Procédé suivant l'une quelconque des revendications précédentes, dans lequel la protéine de liaison peut passer sélectivement à travers les parois des capillaires qui fournissent le sang à une tumeur.

FIG. 1  BIOTIN—$\overset{\overset{O}{\|}}{C}$—$\overset{H}{N}$—$(CH_2)_4$—$\overset{H}{N}$—⬡—$CH_2$—EDTA—M

BLOOD  ECF  TUMOR  FIG. 2

SLOW  STEP 1

RAPID  STEP 2

RAPID  STEP 3

(A)  (B)

FIG. 3

15